# EUROPEAN PATENT SPECIFICATION

(11) **EP 4 311 511 B1**
(45) Date of publication and mention of the grant of the patent: **10.06.2026**
(21) Application number: 23186422.4
(22) Date of filing: 19.07.2023
(51) Int. Cl.: A61B 18/12, A61B 18/14, A61B 18/00, A61N 7/00, A61B 18/18, A61N 7/02

(54) **INTEGRATED CHECK VALVE FOR HEATING ELEMENT COOLING IN OCCLUSIVE DENERVATION CATHETERS**
INTEGRIERTES RÜCKSCHLAGVENTIL ZUR HEIZELEMENTKÜHLUNG IN OKKLUSIVEN DENERVATIONSKATHETERN
CLAPET ANTI-RETOUR INTÉGRÉ POUR LE REFROIDISSEMENT D'ÉLÉMENT CHAUFFANT DANS DES CATHÉTERS DE DÉNERVATION OCCLUSIFS

(30) Priority: 25.07.2022 US 202263391894 P; 12.06.2023 US 202318333274
(43) Date of publication of application: 31.01.2024
(73) Proprietor: Medtronic Ireland Manufacturing Unlimited Company, Dublin 2, D02 T380 (IE)
(72) Inventor: HETTRICK, Douglas A., Dublin, 2 DO2 T380 (IE); COATES, Paul J., Dublin, 2 DO2 T380 (IE); MCCAFFREY, Gerry O., Dublin, 2 DO2 T380 (IE)
(74) Representative: Zimmermann & Partner Patentanwälte mbB

(56) References cited:
- WO-A1-2021/225041
- WO-A1-2023/213650
- WO-A2-2022/067146
- US-A1- 2011 301 587
- US-A1- 2012 232 549
- US-A1- 2014 371 736
- US-A1- 2018 036 071
- US-A1- 2021 162 114

## Description

### BACKGROUND

Vascular denervation (for example, Renal denervation (RDN)) has been shown to lower blood pressure in uncontrolled hypertensive patients using transcutaneous catheter devices delivered into the arteries. Some such catheter devices use treatment elements on the distal end of the catheter to deliver thermal energy (for example, radiofrequency, microwave, and ultrasonic) to a treatment area of an artery to damage targeted nerves. Some designs use ultrasonic energy delivered via piezoelectric crystals. One design of an ultrasonic catheter system applies multiple crystals in a geometric pattern in a non-occlusive basket design. Other designs of ultrasonic catheter systems include a cylindrical crystal positioned in an occlusive balloon. In these ultrasonic designs and in other types of occlusive catheter systems with heating elements, fluid is actively circulated through the balloon to cool the balloon and protect the arterial wall from thermal damage. Such cooling of the arterial wall still allows thermal destruction of nerves in the adventitial extra-arterial space.

Document WO 2021/225041 A1 relates to a catheter system, a catheter equipped with circuit, a circuit and a fluid circulation method.

### SUMMARY OF THE INVENTION

The invention provides a system according to claim 1. Preferred embodiments are defined in the dependent claims.

### SUMMARY OF THE DISCLOSURE

As noted, some vascular denervation catheter devices use ultrasonic, radiofrequency, microwave, and other types of heating elements positioned within or on the surface of an occlusive balloon, through which cooling fluid is circulated during treatment. The capacity to convectively cool the heating element depends on the physical constraints limiting the volume and rate of fluid circulation within the balloon. Therefore, although the ability to control the volume and flow rate of fluid exiting the balloon is important to cooling, it is limited in current device configurations, especially by catheter size (i.e., diameter). Therefore, in some instances, if the heating element becomes too hot, the circulating fluid itself may not be capable of adequate cooling. In other instances, the physical constraints limiting the volume and rate of fluid circulation within the balloon also limit the operation of occlusive radiofrequency catheters, where active cooling is required to ensure adequate control of tissue heating. To address, among other things, these problems, devices, systems, and methods are provided herein for controllably increasing cooling fluid flow rates in occlusive denervation catheters.

Embodiments described herein provide, among other things, occlusive denervation catheters with treatment elements including integrated check valves and varied lumen positioning. The integrated check valve opens at a certain pressure to allowing a specified maximal fluid flow. The check valve enables some (sterile) cooling fluid volume to escape from the balloon into the artery under treatment. Additional fluid flow could still be recovered via the outflow lumen of the treatment element. In some aspects, the check valve is activated by increasing the pressure of the inlet flow during the high-power phase of ultrasonic energy delivery and reducing the pressure after energy delivery. In some aspects, inlet pressure may be increased based on an actual or predicted temperature increase in the balloon.

Some alternative embodiments described herein provide proportional check valves, which allow for the selection or adjustment of the valve opening pressure or degree of valve opening to provide more granular control of cooling fluid flow rates.

Further disclosed herein are occlusive denervation catheters with treatment elements including integrated check valves and varied lumen positioning, wherein an integrated check valve opens at a certain pressure to allowing a specified maximal fluid flow, wherein the check valve enables some (sterile) cooling fluid volume to escape from the balloon into the artery under treatment, wherein additional fluid flow could still be recovered via the outflow lumen of the treatment element, wherein, in some aspects, the check valve is activated by increasing the pressure of the inlet flow during the high-power phase of ultrasonic energy delivery and reducing the pressure after energy delivery, and wherein, in some aspects, inlet pressure may be increased based on an actual or predicted temperature increase in the balloon.

The details of one or more aspects of the disclosure are set forth in the accompanying drawings and the description below. Other features, objects, and advantages of the techniques described in this disclosure will be apparent from the description and drawings, and from the claims.

The methods disclosed herein are not claimed.

### BRIEF DESCRIPTION OF THE DRAWINGS

The accompanying figures, where like reference numerals refer to identical or functionally similar elements throughout the separate views, which together with the detailed description below are incorporated in and form part of the specification and serve to further illustrate various embodiments of concepts that include the claimed embodiments, and to explain various principles and advantages of aspects of those embodiments.
FIG. 1 is schematic illustration of an example vascular denervation system in accordance with some embodiments.
FIG. 2 is a block diagram that illustrates an electronic controller of the system of FIG. 1 in accordance with some embodiments.
FIG. 3A illustrates an example treatment element of the vascular denervation system of FIG. 1 in accordance with some embodiments.
FIG. 3B illustrates an example treatment element of the vascular denervation system of FIG. 1 in accordance with some embodiments.
FIG. 3C is a chart illustrating aspects of the operation of the system of FIG. 1 in accordance with some embodiments.
FIG. 4A illustrates an example treatment element of the vascular denervation system of FIG. 1 in accordance with some embodiments.
FIG. 4B illustrates an example treatment element of the vascular denervation system of FIG. 1 in accordance with some embodiments.
FIG. 4C is a chart illustrating aspects of the operation of the system of FIG. 1 in accordance with some embodiments.
FIG. 5A illustrates an example treatment element of the vascular denervation system of FIG. 1 in accordance with some embodiments.
FIG. 5B is a chart illustrating aspects of the operation of the system of FIG. 1 in accordance with some embodiments.
FIG. 6 illustrates an example treatment element of the vascular denervation system of FIG. 1 in accordance with some embodiments.

Skilled artisans will appreciate that elements in the figures are illustrated for simplicity and clarity and have not necessarily been drawn to scale. For example, the dimensions of some of the elements in the figures may be exaggerated relative to other elements to help to improve understanding of embodiments of the present invention.

The apparatus and method components have been represented where appropriate by conventional symbols in the drawings, showing only those specific details that are pertinent to understanding the embodiments of the present invention so as not to obscure the disclosure with details that will be readily apparent to those of ordinary skill in the art having the benefit of the description herein.

### DETAILED DESCRIPTION

Before any embodiments of the invention are explained in detail, it is to be understood that the invention is not limited in its application to the details of construction and the arrangement of components set forth in the following description or illustrated in the following drawings. The invention is capable of other embodiments and of being practiced or of being carried out in various ways, and is limited only by the scope of the appended claims.

Also, it is to be understood that the phraseology and terminology used herein is for the purpose of description and should not be regarded as limiting. The terms "mounted," "connected," and "coupled" are used broadly and encompass both direct and indirect mounting, connecting, and coupling. The terms "connected" and "coupled" are not restricted to physical or mechanical connections or couplings, and can include electrical connections or couplings, whether direct or indirect. Electronic communications and notifications described herein may be performed using any known or future-developed means including wired connections, wireless connections, etc.

For ease of description, some or all of the example systems presented herein are illustrated with a single exemplar of each of its component parts. Some examples may not describe or illustrate all components of the systems. Other embodiments may include more or fewer of each of the illustrated components, may combine some components, or may include additional or alternative components.

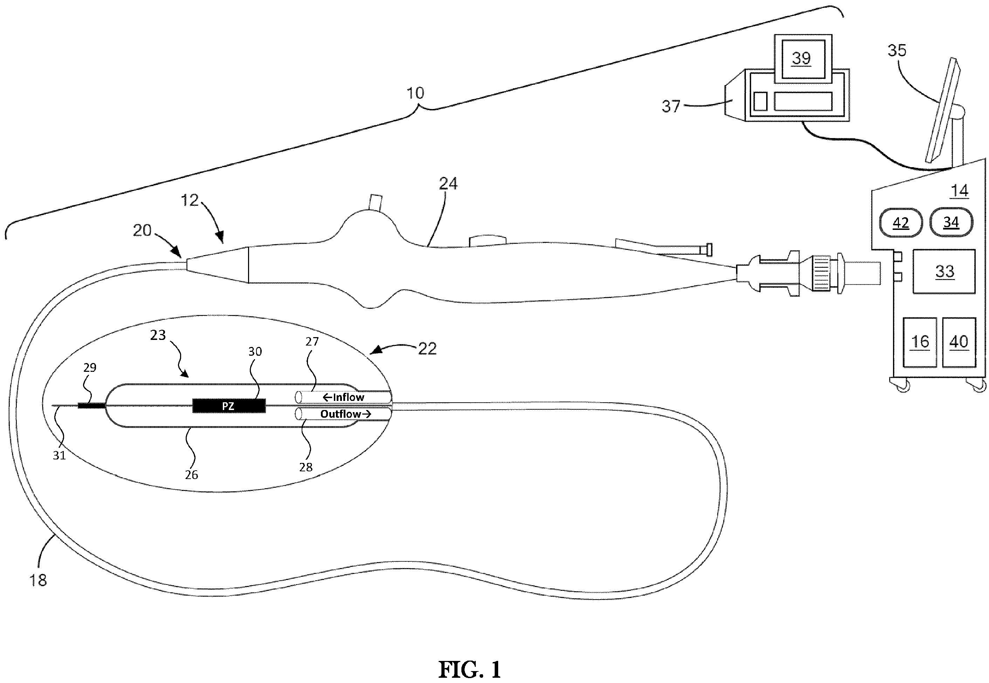

FIG. 1 illustrates an example system 10 that is suitable for performing vascular denervation. The system 10 is for delivering and applying thermal energy to an area of target tissue, such as, for example, an area of tissue within the hepatic artery to cause denervation. The system 10 may be configured to perform denervation in the renal arteries, as well as in other vessels, including the celiac trunk and its branches, the splenic artery and its branches, the common hepatic artery and its branches, the left gastric artery and its branches, and the superior and inferior mesenteric arteries and their branches. The system 10 may also be configured to perform denervation two or more vessels (e.g., in the renal artery and the common hepatic artery and its branches).

The system 10 may generally include a treatment device, such as a medical device 12, and a control unit 14 for operating, monitoring, and regulating the operation of the device 12 (for example, with the electronic controller 33), and a fluid supply reservoir 16 for delivering fluid (for example, a sterile coolant) to the device 12.

The medical device 12 is a highly flexible treatment device that is suitable for passage through the vasculature. The device 12 may be adapted for use with the fluid supply reservoir 16 to denervate portions of an artery. In the example illustrated, the device 12 has an elongate body 18 having a proximal portion 20 and a distal portion 22. The distal portion 22 includes a treatment element 23. The proximal portion 20 of the device 12 is mated to a handle 24 that can include an element such as a lever or knob for manipulating the elongate body 18 and the treatment element 23. The distal portion 22 may also define a distal tip 29 defining an aperture (not shown) sized to allow for the passage of a guidewire 31 through the elongate body 18 and through the aperture.

The elongate body 18 is sized and configured to be passable through a patient's vasculature and/or positionable proximate to the area of target tissue, and may include one or more lumens (for example, the inflow lumen 27 and the outflow lumen 28) disposed within the elongate body 18 that provide mechanical, electrical, and/or fluid communication between the proximal portion 20 of the elongate body 18 and the distal portion 22 of the elongate body 18. In some aspects, the elongate body 18 includes a guidewire lumen through which a sensing device, mapping device, the guidewire 31, or other system component may be located and extended from the distal portion 22 of the medical device 12. The elongate body 18 may be rigid and/or flexible to facilitate the navigation of the device 12 within a patient's body. In one aspect, the distal portion 22 of the elongate body 18 is flexible to allow for more desirable positioning proximate to an area of target tissue (e.g., positioning within a renal artery, a hepatic artery, the splanchnic bed, the pulmonary artery, the aortic root, the carotid body, and the like). To access an area of target tissue, the device 12 may be inserted through one or more blood vessels, such as, for example, one or more brachial arteries, one or more radial arteries, one or more femoral arteries, or other points of access including venous access (e.g., for greater splanchnic nerve denervation or through the jugular vein for carotid body ablation).

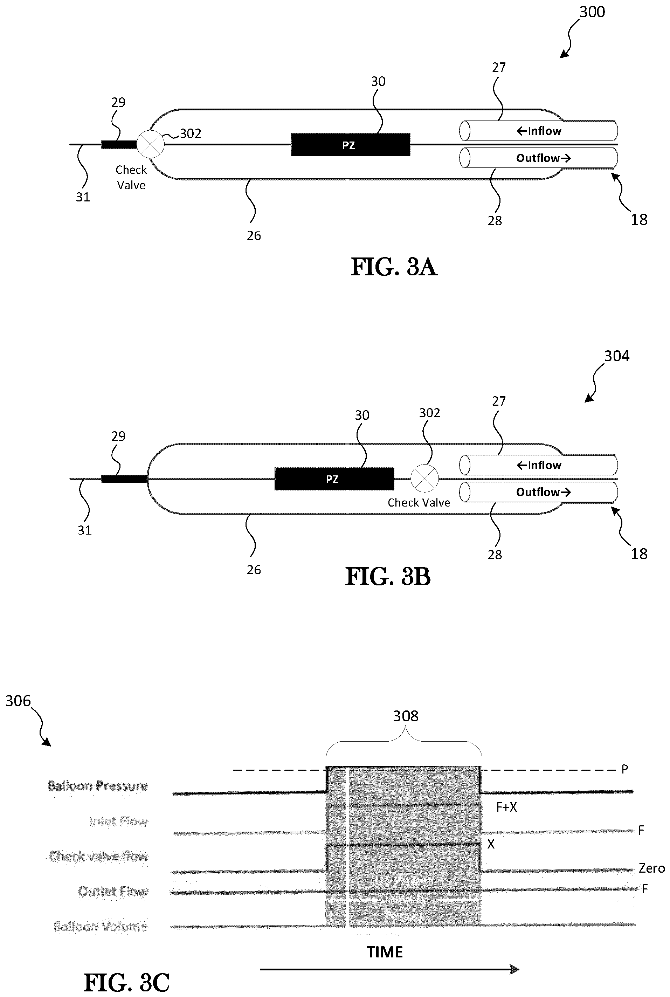

The treatment element 23 includes an expandable element 26 (for example, an occlusive balloon), through which fluid from the fluid supply reservoir 16 is circulated. A fluid inflow lumen 27 is in fluid communication with the fluid supply reservoir 16 in the console 14 to supply fluid to cool the thermal element 30 in response to console commands and other control input. In some aspects, a vacuum pump 34 (electronically coupled to and controlled by the electronic controller 33) in the console 14 creates a low pressure environment in an outflow lumen 28 so that the fluid is drawn into the outflow lumen 28, away from the expandable element 26, towards the proximal portion 20 of the elongate body 18, and into the fluid recovery reservoir 40 within the console 14. When expanded, the expandable element 26 is sized and configured to fit within an area of the artery under treatment such that the thermal element 30 is substantially centered within the artery. As described herein, some aspects of the treatment element 23 (for example, as illustrated in FIGS. 3A, 3B, 4A, 4B, 5A, and 5B) include one or more check valves, which allow sterile fluid to escape the expandable element 26 (for example, through the distal tip 29) into the artery under treatment.

Although not illustrated in FIG. 1, in some aspects, the treatment element 23 includes one or more temperature sensors positioned on or in the treatment element to continuously measure temperature values within, on, or proximate to the expandable element 26. The one or more temperature sensors transmit temperature signals to the electronic controller 33 of the console 14.

In some aspects, the console 14 includes one or more pressure sensors 42 to continuously record the instantaneous pressure values within the expandable element 26. The pressure sensors 42 may then generate and transmit a pressure signal to the electronic controller 33 of the console 14. Although not illustrated in FIG. 1, in some aspects, the device 12 may also include a pressure sensor 42 within the expandable element 26 (not shown) or a pressure monitoring tube (enclosed in the elongate body 18) in fluid communication with the pressure sensor 42 (housed in the console 14) and the expandable element 26.

In some aspects, the system 10 may also include the use of one or more flow sensors (not shown) to monitor how much fluid is flowing into the expandable element 26. The flow sensor may be positioned to sense flow in the inflow lumen 27 or the outflow lumen 28 and is configured to measure the rate or speed of fluid at a certain location and report the rate or speed to the electronic controller 33. In some aspects, each of the inflow lumen 27 or the outflow lumen 28 is monitored by a dedicated flow sensor.

The treatment element 23 also includes at least one thermal element 30 for applying thermal energy to target tissue to accomplish denervation. The thermal element 30 may deliver thermal energy via radiofrequency energy, microwave energy, ultrasonic energy, or another suitable energy type. In the illustrated example, the thermal element 30 is a piezoelectric ultrasound transducer positioned within the expandable element 26. The thermal element 30 is in electrical communication with the control unit 14 or other external ultrasound control unit such that the piezoelectric crystal rapidly vibrates when electric current is applied to the piezoelectric ultrasound transducer. The vibrations of each crystal generate sound waves at ultrasound frequencies that may be delivered to the area of target tissue. In some aspects, the thermal element 30 is configured to deliver ultrasonic energy circumferentially. As the thermal element 30 operates, it is cooled with fluid circulating through the expandable element 26, as described herein. It should also be noted that, although the example illustrated in FIG. 1 depicts a single thermal element 30 located on the centerline of the treatment element 23, one or more thermal element(s) might be mounted on an outside or inside surface of the expandable element 26, or any other suitable location of the treatment element 23. For example, in some instances, the treatment element 23 includes multiple linear transducers disposed to achieve circumferential energy delivery.

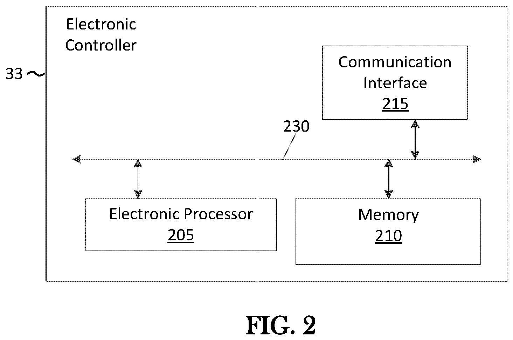

In the illustrated example, the control unit 14 includes an electronic controller 33 (described more particularly with respect to FIG. 2) programmed or programmable to execute the automated or semi-automated operation and performance of the features, sequences, calculations, or procedures described herein. The electronic controller 33 is communicatively coupled to the temperature sensors, pressure sensors, flow sensors, and other components of the device 12, including the vacuum pump 34 and the thermal element 30. In some instances, the control unit 14 includes a separate electronic controller to control the thermal element 30 for energy delivery. In such instances, the electronic controllers are in communication to deliver energy and control temperature via coolant flow. In some instances, a separate energy delivery electronic controller also receives sensor feedback (e.g., temperature and pressure readings) as well as coolant flow information and responds to this input by adjusting the energy delivery as described herein. The control unit 14 may include one or more user input devices, controllers, speakers, and/or electronic displays 35 (each coupled to and controllable by the electronic controller 33) for collecting and conveying information from and to the user.

In some aspects, the treatment element 23 includes ultrasonic transducers (not shown) to record the reflected, refracted, scattered, and/or attenuated ultrasound signals from the target tissue. As the transducers record the ultrasound signals, they begin to vibrate and the mechanical vibrations are converted into electric current signals that are transmitted back to the control unit 14 or an external ultrasound control unit 37 which processes the signals to generate a sonogram or ultrasonogram showing the patient's tissue, organs, and/or a location of the device 12 within the patient's body. The sonogram may then be relayed to a clinician via a display 35 of the control unit 14, or via a display 39 of the external ultrasound control unit 37, to assist the physician in positioning the device 12 near or proximate to a desired treatment location.

FIG. 2 illustrates an example embodiment of the electronic controller 33, which includes an electronic processor 205 (for example, a microprocessor, application specific integrated circuit, etc.), a memory 210, and an input/output interface 215. The memory 210 may be made up of one or more non-transitory computer-readable media and includes at least a program storage area and a data storage area. The program storage area and the data storage area can include combinations of several types of memory, such as read-only memory ("ROM"), random access memory ("RAM") (for example, dynamic RAM ("DRAM"), synchronous DRAM ("SDRAM"), etc.), electrically erasable programmable read-only memory ("EEPROM"), flash memory, or other suitable memory devices. The electronic processor 205 is coupled to the memory 210 and the input/output interface 215. The electronic processor 205 sends and receives information (for example, from the memory 210 and/or the input/output interface 215) and processes the information by executing one or more software instructions or modules, capable of being stored in the memory 210, or another non-transitory computer readable medium. The software can include firmware, one or more applications, program data, filters, rules, one or more program modules, and other executable instructions. The electronic processor 205 is configured to retrieve from the memory 210 and execute, among other things, software for performing methods as described herein.

The input/output interface 215 transmits and receives information from devices external to the electronic controller 33 (for example, over one or more wired and/or wireless connections), for example, components of the system 10. The input/output interface 215 receives input (for example, from a human machine interface of the console 14), provides system output or a combination of both. The input/output interface 215 may also include other input and output mechanisms, which for brevity are not described herein and which may be implemented in hardware, software, or a combination of both.

It should be understood that although FIG. 2 illustrates only a single electronic processor 205, memory 210, and input/output interface 215, alternative embodiments of the electronic controller 33 may include multiple processors, memory modules, and/or input/output interfaces. It should also be noted that the system 10 may include other electronic controllers, each including similar components as, and configured similarly to, the electronic controller 33. In some embodiments, the electronic controller 33 is implemented partially or entirely on a semiconductor (for example, a field-programmable gate array ["FPGA"] semiconductor) chip. Similarly, the various modules and controllers described herein may be implemented as individual controllers, as illustrated, or as components of a single controller. In some embodiments, a combination of approaches may be used.

FIG. 3A illustrates an example 300 of the treatment element 23. Treatment element 300 includes, in addition to the components illustrated and described above, a check valve 302. The check valve 302 is configured to open and remain open while a fluid pressure within in the expandable element 26 meets or exceeds a threshold pressure value and to close and remain closed when the fluid pressure within in the expandable element 26 is below the threshold pressure value. In the illustrated example, the check valve 302 is positioned on the treatment element 23 such that when the check valve 302 is open, the fluid pressure within the expandable element 26 causes fluid to flow through the check valve 302, out of the distal tip 29, and into the artery under treatment. FIG. 3B illustrates an example 304 of the treatment element 23. The treatment element 304 is similar to the treatment element 300, except that the check valve 302 is positioned on an exterior surface of the expandable element 26.

During use of the treatment element 300 (or the treatment element 304), the pressure of the fluid in the expandable element 26 may be increased to trigger the check valve 302 and increase fluid flow. For example, as illustrated in the chart 306 of FIG. 3C, the electronic controller 33 controls (energizes) the thermal element 30 to deliver ultrasonic (US) power to the target tissue during a delivery period 308. During the delivery period 308, the electronic controller 33 controls the pump 34 to increase the balloon pressure (pressure within the expandable element 26) above a pressure threshold P, causing the check valve 302 to open, raising the check flow valve fluid flow rate from 0 to X. This, in turn causes the inlet flow rate to increase from F (matching the outflow rate when the check valve 302 is closed) to F+X, increasing the overall fluid flow through the expandable element 26, which provides more cooling to the thermal element 30 while maintaining a constant volume in the expandable element 26.

In some instances, as described above, the electronic controller 33 controls the thermal element 30 to deliver energy to the tissue, while simultaneously controlling the pump 34 to increase the balloon pressure (pressure within the expandable element 26) above the pressure threshold P to open the check valve 302 throughout the delivery period 308. In some instances, the electronic controller 33 is configured to control the pump 34 to increase fluid flow during the delivery period 308 based on other factors. For example, in some instances, the electronic controller 33 controls the vacuum pump based on an actual or predicted temperature for the treatment element 23. The electronic controller 33 may receive temperature readings from a temperature sensor situated at the distal end 22, compare those readings to a threshold value, and control the pump 34 to increase the balloon pressure to open the check valve 302 when the current temperature reading exceeds or is within a range of the threshold. In some instances, the electronic controller 33 may control the pump 34 to increase the balloon pressure when a predicted temperature (e.g., based on a calculated rate of change for the temperature readings) exceeds or is within a range of a threshold value. In some instances, the electronic controller 33 may control the pump 34 to increase the balloon pressure when a rate of change for the temperature readings exceeds or is within a range of a threshold value.

In some instances, the electronic controller 33 is configured to control the thermal element 30 and other aspects of treatment based on the temperature of the fluid being expelled (or which could potentially be expelled) via the check valve(s). In such instances, the electronic controller 33 is configured to control the thermal element 30 to reduce its energy emissions in response to a temperature reading for the fluid in the expandable element 26 exceeding a fluid temperature threshold value. In some instances, the electronic controller 33 is configured to completely de-energize the thermal element 30 to stop its energy emissions in response to a temperature reading for the fluid in the expandable element 26 exceeding the fluid temperature threshold value.

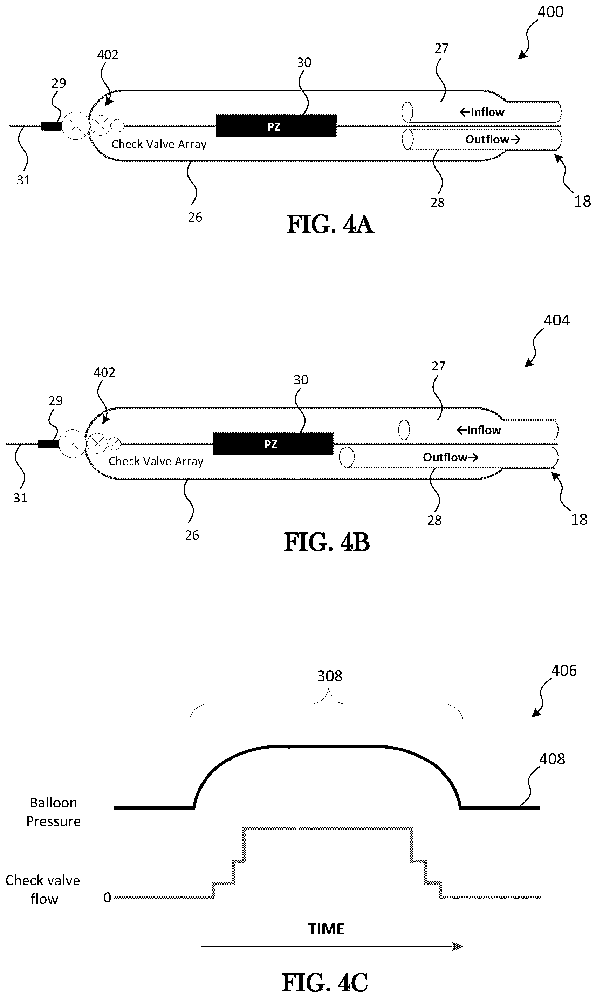

FIG. 4A illustrates an example 400 of the treatment element 23. Treatment element 400 includes, in addition to the components described above, a check valve array 402. As illustrated, the check valve array includes three check valves positioned on the treatment element 23 such that when the any of the check valves in the check valve array 402 is open, the fluid pressure within the expandable element 26 causes fluid to flow through the open check valves, out of the distal tip 29, and into the artery under treatment. In alternative aspects, the check valve array 402 may include fewer or more than three check valves, which may be positioned differently on the treatment element 23. In some aspects, each of the check valves in the check valve array 402 has a different opening pressure threshold, such that escalating pressure may open the check valves progressively to increase the flow of fluid out of the expandable element 26. In some aspects, each of the check valves in the check valve array 402 has a different opening diameter.

FIG. 4B illustrates a variant 404 of the treatment element 400. The treatment element 404 is similar to the treatment element 400, except that the outflow lumen 28 is offset inward compared to the position of the inflow lumen 27. Positioning the outflow lumen 28 closer to the thermal element 30 results in heated fluid being pulled more quickly from the expandable element 26.

The check valves of the check valve array 402 may be configured with various pressure thresholds and opening diameters to produce a variety of fluid flows over a range of fluid pressures. For example, as illustrated in the chart 406 of FIG. 4C, ultrasonic (US) power is delivered to the target tissue (via the thermal element 30) during a delivery period 308. During the delivery period 308, the balloon pressure (pressure within the expandable element 26) is controlled as illustrated by line 408. As a result, the check valve flow increases from and returns to 0 over time in increments proportional to the opening pressures and diameters of the check valves making up the check valve array 402. This allows the fluid flow through the expandable element 26 to be regulated in a varied but predictable way.

In embodiments including multiple check valves configured to open at different pressure thresholds (as described above with respect to FIGS. 4A-C), temperature control may be achieved through determining a different temperature threshold value for each check valves pressure threshold. For example, the electronic controller 33 may control the pump 34 to increase the balloon pressure to a first pressure to trigger a first check valve when the current temperature reading exceeds or is within a range of a first threshold and control the pump 34 to increase the balloon pressure to a second pressure to trigger a second check valve when the current temperature reading exceeds or is within a range of a second threshold.

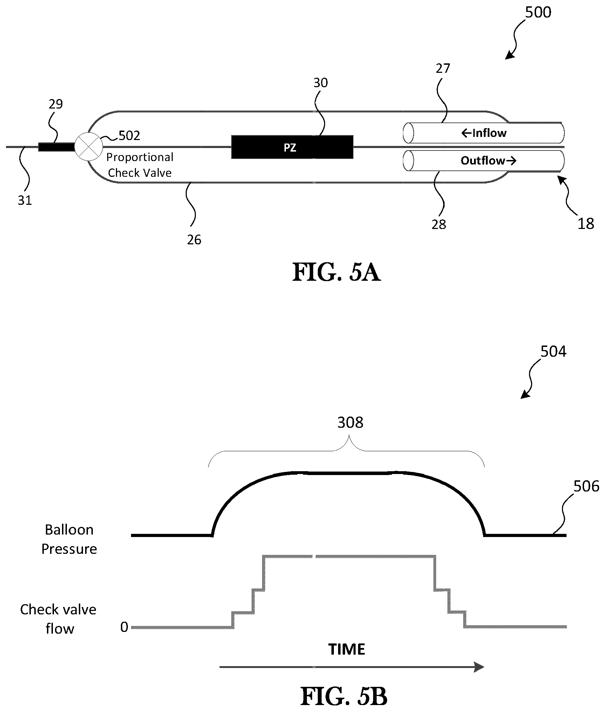

FIG. 5A illustrates an example 500 of the treatment element 23. Treatment element 500 includes, in addition to the components described above, a proportional check valve 502. A proportional check valve opens over a range of opening sizes (for example, diameters) proportional to the fluid pressure within the expandable element 26. As the pressure increases beyond the opening threshold pressure, the proportional check valve 500 allow proportionally more fluid to flow through the open check valve 502, out of the distal tip 29, and into the artery under treatment. The check valves of the check valve array 402 may be configured with various pressure thresholds and opening diameters to produce a variety of fluid flows over a range of fluid pressures. For example, as illustrated in the chart 504 of FIG. 5C, ultrasonic (US) power is delivered to the target tissue (via the thermal element 30) during a delivery period 308. During the delivery period 308, the balloon pressure (pressure within the expandable element 26) is controlled as illustrated by line 506. As a result, the check valve flow increases from and returns to 0 over time in increments proportional to the fluid pressure applied to the proportional check valve 502. This allows the fluid flow through the expandable element 26 to be regulated in a varied but predictable way.

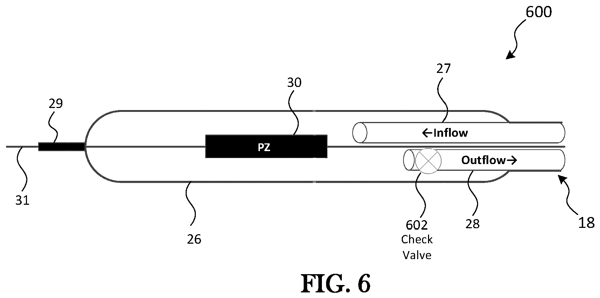

FIG. 6 illustrates an example 600 of the treatment element 23. Treatment element 300 includes, in addition to the components illustrated and described above, a check valve 602. The check valve 602 is configured to open and remain open while a fluid pressure within in the expandable element 26 meets or exceeds a threshold pressure value and to close and remain closed when the fluid pressure within in the expandable element 26 is below the threshold pressure value. In the illustrated example, the check valve 602 is positioned within the expandable element 26 on the outflow lumen. In the illustrated example, the check valve 602 may remain partially open when the fluid pressure is below a threshold, such that the fluid flow from the inflow lumen 27 and the fluid flow out of the outflow lumen 28 are substantially equal. In one aspect, when the check valve 602 is open, the fluid pressure within the expandable element 26 causes check valve 602 to increase its opening size and increase fluid flow through the outflow lumen 28. In some aspects, the outflow lumen 28 may have an opening narrower than its body to restrict fluid flow unless a check valve 602 positioned on the body of the outflow lumen 28 is opened to provide a second opening in the outflow lumen 28, increasing the fluid flow out of the expandable element 26. FIG. 6 illustrates an aspect of the treatment element 23 where the inflow lumen 27 is offset inward compared to the position of the outflow lumen 28. Positioning the inflow lumen 27 closer to the thermal element 30 results in cooler fluid reaching the thermal element 30 more quickly to ensure better cooling throughout the expandable element 26.

In the foregoing specification, specific embodiments are described. However, one of ordinary skill in the art appreciates that various modifications and changes may be made without departing from the scope of the invention as set forth in the claims below. Accordingly, the specification and figures are to be regarded in an illustrative rather than a restrictive sense, and all such modifications are intended to be included within the scope of present teachings. For example, while some embodiments are illustrated and described as including a single ultrasonic energy source, such embodiments could be applied to any balloon-based system, including those with other types or quantities of heating element energy sources.

It should also be noted that a plurality of hardware and software-based devices, as well as a plurality of different structural components may be utilized to implement the embodiments provided herein. It should also be noted that a plurality of hardware and software-based devices, as well as a plurality of different structural components may be used to implement the invention. In addition, it should be understood that embodiments may include hardware, software, and electronic components or modules that, for purposes of discussion, may be illustrated and described as if the majority of the components were implemented solely in hardware. However, one of ordinary skill in the art, and based on a reading of this detailed description, would recognize that, in at least one embodiment, the electronic based aspects of the invention may be implemented in software (e.g., stored on non-transitory computer-readable medium) executable by one or more processors. As such, it should be noted that a plurality of hardware and software-based devices, as well as a plurality of different structural components may be utilized to implement the invention. For example, "control units" and "controllers" described in the specification can include one or more processors, one or more application specific integrated circuits (ASICs), one or more memory modules including non-transitory computer-readable media, one or more input/output interfaces, and various connections (e.g., a system bus) connecting the components.

It will be appreciated that some embodiments may be comprised of one or more electronic processors such as microprocessors, digital signal processors, customized processors, and field programmable gate arrays (FPGAs) and unique stored program instructions (including both software and firmware) that control the one or more processors to implement, in conjunction with certain non-processor circuits, some, most, or all of the functions of the method and/or apparatus described herein. Alternatively, some or all functions could be implemented by a state machine that has no stored program instructions, or in one or more application specific integrated circuits (ASICs), in which each function or some combinations of certain of the functions are implemented as custom logic. Of course, a combination of the two approaches could be used.

Moreover, some embodiments may be implemented as a computer-readable storage medium having computer readable code stored thereon for programming a computer (for example, comprising an electronic processor) to perform a method as described and claimed herein. Examples of such computer-readable storage media include, but are not limited to, a hard disk, a CD-ROM, an optical storage device, a magnetic storage device, a ROM (Read Only Memory), a PROM (Programmable Read Only Memory), an EPROM (Erasable Programmable Read Only Memory), an EEPROM (Electrically Erasable Programmable Read Only Memory) and a Flash memory. Further, it is expected that one of ordinary skill, notwithstanding possibly significant effort and many design choices motivated by, for example, available time, current technology, and economic considerations, when guided by the concepts and principles disclosed herein will be readily capable of generating such software instructions and programs and ICs with minimal experimentation.

It should be understood that although certain drawings illustrate hardware and software located within particular devices, these depictions are for illustrative purposes only. In some examples, the illustrated components may be combined or divided into separate software, firmware and/or hardware. For example, instead of being located within and performed by a single electronic processor, logic and processing may be distributed among multiple electronic processors. Regardless of how they are combined or divided, hardware and software components may be located on the same computing device or may be distributed among multiple different devices.

In this specification, relational terms such as first and second, top and bottom, and the like may be used solely to distinguish one entity or action from another entity or action without necessarily requiring or implying any actual such relationship or order between such entities or actions. The terms "comprises," "comprising," "has," "having," "includes," "including," "contains," "containing," or any other variation thereof, are intended to cover a non-exclusive inclusion, such that a process, method, article, or apparatus that comprises, has, includes, contains a list of elements does not include only those elements but may include other elements not expressly listed or inherent to such process, method, article, or apparatus. An element proceeded by "comprises ...a," "has ... a," "includes ...a," or "contains ...a" does not, without more constraints, preclude the existence of additional identical elements in the process, method, article, or apparatus that comprises, has, includes, contains the element. The terms "a" and "an" are defined as one or more unless explicitly stated otherwise herein. The terms "substantially," "essentially," "approximately," "about," or any other version thereof, are defined as being close to as understood by one of ordinary skill in the art, and in one nonlimiting embodiment the term is defined to be within 10%, in another embodiment within 5%, in another embodiment within 1% and in another embodiment within 0.5%. A device or structure that is "configured" in a certain way is configured in at least that way but may also be configured in ways that are not listed.

Various features and advantages of the embodiments presented herein are set forth in the following claims.

## Claims

1. A system (10) for ablating tissue, the system (10) comprising:
a treatment device including a treatment element (23), the treatment element (23) including an expandable element (26), a thermal element (30), an inflow lumen (27) for providing a fluid to the expandable element (26), and a check valve (302) for controllably allowing the fluid to flow from the expandable element (26); and
one or more electronic controllers (33) coupled to the treatment device and configured to:
control a pump to provide a volume of the fluid to the expandable element (26) via the inflow lumen (27) at a first pressure;
control the thermal element (30) to emit energy to perform an ablation on a target tissue during a power delivery period; and
during the power delivery period, control the pump to provide the volume of the fluid to the expandable element (26) via the inflow lumen (27) at a second pressure higher than the first pressure, wherein the check valve (302) is configured such that the second pressure causes the check valve (302) to open.

2. The system (10) of claim 1, wherein the one or more electronic controllers (33) are further configured to:
responsive to an expiration of the power delivery period, control the thermal element (30) to stop emitting energy; and
responsive to the expiration of the power delivery period, control the pump to provide the volume of fluid to the expandable element (26) via the inflow lumen (27) at the first pressure.

3. The system (10) of any of the preceding claims, wherein the treatment device further includes:
a temperature sensor; and
wherein the one or more electronic controllers (33) are coupled to the temperature sensor and further configured to, during the power delivery period:
receive a temperature reading from the temperature sensor; and
control the pump based on the temperature reading.

4. The system (10) of claim 3, wherein the one or more electronic controllers (33) are further configured to:
compare the temperature reading to a threshold value; and
control the pump based on the temperature reading by controlling the pump to provide the volume of the fluid to the expandable element (26) via the inflow lumen (27) at the second pressure only when the temperature reading exceeds the threshold value; and/or wherein the one or more electronic controllers (33) are further configured to:
in response to the temperature reading exceeding a fluid temperature threshold value during the power delivery period, control the thermal element (30) to stop emitting energy; and/or wherein the one or more electronic controllers (33) are further configured to:
receive a second temperature reading from the temperature sensor;
determine a predicted temperature based on the first and second temperature readings;
compare the predicted temperature to a second threshold value; and
control the pump based on the temperature reading by controlling the pump to provide the volume of the fluid to the expandable element (26) via the inflow lumen (27) at the second pressure only when the predicted temperature exceeds the second threshold value.

5. The system (10) of any of claims 3-4, wherein the one or more electronic controllers (33) are further configured to, during the power delivery period:
receive a second temperature reading from the temperature sensor;
determine a rate of change based on the first and second temperature readings;
compare the rate of change to a second threshold value; and
control the pump based on the temperature reading by controlling the pump to provide the volume of the fluid to the expandable element (26) via the inflow lumen (27) at the second pressure only when the rate of change is within a predetermined range of the second threshold value.

6. The system (10) of any of the preceding claims, wherein the check valve (302) is configured to, when open, increase a fluid flow through the expandable element (26) while maintaining the volume of the fluid within the expandable element (26); and/or wherein the check valve (302) is positioned on the treatment element (23) such that when the check valve (302) is open, a fluid pressure within the expandable element (26) causes the fluid to flow through the check valve (302) out of a distal tip of the treatment element (23); and/or wherein the check valve (302) is positioned on an exterior surface of the expandable element (26) such that when the check valve (302) is open, a fluid pressure within the expandable element (26) causes the fluid to flow through the check valve (302) out of the treatment element (23).

7. The system (10) of any of the preceding claims, wherein the treatment element (23) includes a plurality of check valves (302) positioned on the treatment element (23) such that when any of the plurality of check valves (302) is open, a fluid pressure within the expandable element (26) causes the fluid to flow out of a distal tip of the treatment element (23).

8. The system (10) of claim 7, wherein each of the plurality of check valves (302) is configured to open at a different pressure threshold, such that a fluid flow through the expandable element (26) is proportional to a fluid pressure applied by the pump, or wherein each of the plurality of check valves (302) is configured with a different opening size and to open at a different pressure threshold, such that a fluid flow through the expandable element (26) is proportional to a fluid pressure applied by the pump.

9. The system (10) of any of the preceding claims, wherein:
the treatment element (23) further includes an outflow lumen for removing fluid from the expandable element (26), and
one of the inflow lumen (27) and the outflow lumen is positioned more closely, with respect to the other, to the thermal element (30).

10. The system (10) of any of the preceding claims, wherein the check valve (302) is configured to open at a plurality of opening sizes corresponding to a plurality of pressure thresholds, such that a fluid flow through the expandable element (26) is proportional to a fluid pressure applied by the pump.

11. The system (10) of any of the preceding claims, wherein:
the treatment element (23) further includes an outflow lumen for removing fluid from the expandable element (26), and
the check valve (302) is positioned at an opening of the outflow lumen.

## Patentansprüche

1. System (10) zur Ablation von Gewebe, wobei das System (10) umfasst:
eine Behandlungsvorrichtung, die ein Behandlungselement (23) aufweist, wobei das Behandlungselement (23) ein expandierbares Element (26), ein thermisches Element (30), ein Zuflusslumen (27), um dem expandierbaren Element (26) ein Fluid bereitzustellen, und ein Rückschlagventil (302) aufweist, um steuerbar zuzulassen, dass das Fluid aus dem expandierbaren Element (26) fließt; und
eine oder mehrere elektronische Steuerungen (33), die mit der Behandlungsvorrichtung gekoppelt und dazu ausgelegt sind:
eine Pumpe dahingehend zu steuern, dem expandierbaren Element (26) ein Volumen des Fluids über das Zuflusslumen (27) mit einem ersten Druck bereitzustellen;
das thermische Element (30) dahingehend zu steuern, Energie zu emittieren, um eine Ablation an einem Zielgewebe während eines Stromlieferzeitraums durchzuführen; und
während des Stromlieferzeitraums die Pumpe dahingehend zu steuern, dem expandierbaren Element (26) das Volumen des Fluids über das Zuflusslumen (27) mit einem zweiten Druck bereitzustellen, der höher ist als der erste Druck, wobei das Rückschlagventil (302) derart ausgelegt ist, dass der zweite Druck bewirkt, dass sich das Rückschlagventil (302) öffnet.

2. System (10) nach Anspruch 1, wobei die eine oder die mehreren Steuerungen (33) ferner dazu ausgelegt sind:
in Reaktion auf ein Ablaufen des Stromlieferzeitraums das thermische Element (30) dahingehend zu steuern, das Emittieren von Energie zu beenden; und
in Reaktion auf das Ablaufen des Stromlieferzeitraums, die Pumpe dahingehend zu steuern, dem expandierbaren Element (26) das Volumen des Fluids über das Zuflusslumen (27) mit dem ersten Druck bereitzustellen.

3. System (10) nach einem der vorhergehenden Ansprüche, wobei die Behandlungsvorrichtung ferner aufweist:
einen Temperatursensor; und
wobei die eine oder die mehreren elektronischen Steuerungen (33) mit dem Temperatursensor gekoppelt und ferner dazu ausgelegt sind, während des Stromlieferzeitraums:
einen Temperaturmesswert von dem Temperatursensor zu empfangen; und
die Pumpe basierend auf dem Temperaturmesswert zu steuern.

4. System (10) nach Anspruch 3, wobei die eine oder die mehreren Steuerungen (33) ferner dazu ausgelegt sind:
den Temperaturmesswert mit einem Schwellenwert zu vergleichen; und
die Pumpe basierend auf dem Temperaturmesswert zu steuern durch Steuern der Pumpe dahingehend, dem expandierbaren Element (26) das Volumen des Fluids über das Zuflusslumen (27) mit dem zweiten Druck nur bereitzustellen, wenn der Temperaturmesswert den Schwellenwert übersteigt;
und/oder wobei die eine oder die mehreren elektronischen Steuerungen (33) ferner dazu ausgelegt sind:
in Reaktion darauf, dass der Temperaturmesswert einen Fluidtemperaturschwellenwert während des Stromlieferzeitraums übersteigt, das thermische Element (30) dahingehend zu steuern, das Emittieren von Energie zu beenden; und/oder wobei die eine oder die mehreren elektronischen Steuerungen (33) ferner dazu ausgelegt sind:
einen zweiten Temperaturmesswert von dem Temperatursensor zu empfangen;
eine vorhergesagte Temperatur basierend auf dem ersten und dem zweiten Temperaturmesswert zu bestimmen;
den vorhergesagten Temperaturmesswert mit einem zweiten Schwellenwert zu vergleichen; und
die Pumpe basierend auf dem Temperaturmesswert zu steuern durch Steuern der Pumpe dahingehend, dem expandierbaren Element (26) das Volumen des Fluids über das Zuflusslumen (27) mit dem zweiten Druck nur bereitzustellen, wenn der Temperaturmesswert den Schwellenwert übersteigt.

5. System (10) nach einem der Ansprüche 3-4, wobei die eine oder die mehreren elektronischen Steuerungen (33) ferner dazu ausgelegt sind, während des Stromlieferzeitraums:
einen zweiten Temperaturmesswert von dem Temperatursensor zu empfangen;
eine Änderungsrate basierend auf dem ersten und dem zweiten Temperaturmesswert zu bestimmen;
die Änderungsrate mit einem zweiten Schwellenwert zu vergleichen; und
die Pumpe basierend auf dem Temperaturmesswert zu steuern durch Steuern der Pumpe dahingehend, dem expandierbaren Element (26) das Volumen des Fluids über das Zuflusslumen (27) mit dem zweiten Druck nur bereitzustellen, wenn die Änderungsrate in einem vorbestimmten Bereich des zweiten Schwellenwerts liegt.

6. System (10) nach einem der vorhergehenden Ansprüche, wobei das Rückschlagventil (302) dazu ausgelegt ist, wenn offen, einen Fluidstrom durch das expandierbare Element (26) zu erhöhen und dabei das Volumen des Fluids innerhalb des expandierbaren Elements (26) beizubehalten; und/oder wobei das Rückschlagventil (302) derart an dem Behandlungselement (23) positioniert ist, dass, wenn das Rückschlagventil (302) offen ist, ein Fluiddruck innerhalb des expandierbaren Elements (26) bewirkt, dass das Fluid durch das Rückschlagventil (302) aus einer distalen Spitze des Behandlungselements (23) herausfließt; und/oder wobei das Rückschlagventil (302) derart an einer Außenseite des expandierbaren Elements (26) positioniert ist, dass, wenn das Rückschlagventil (302) offen ist, ein Fluiddruck innerhalb des expandierbaren Elements (26) bewirkt, dass das Fluid durch das Rückschlagventil (302) aus dem Behandlungselement (23) herausfließt.

7. System (10) nach einem der vorhergehenden Ansprüche, wobei das Behandlungselement (23) eine Vielzahl von Rückschlagventilen (302) aufweist, die derart an dem Behandlungselement (23) positioniert sind, dass, wenn ein beliebiges der Vielzahl von Rückschlagventilen (302) offen ist, ein Fluiddruck innerhalb des expandierbaren Elements (26) bewirkt, dass das Fluid aus einer distalen Spitze des Behandlungselements (23) herausfließt.

8. System (10) nach Anspruch 7, wobei jedes der Vielzahl von Rückschlagventilen (302) dazu ausgelegt ist, sich bei einem anderen Druckschwellenwert zu öffnen, so dass ein Fluidfluss durch das expandierbare Element (26) zu einem von der Pumpe aufgebrachten Fluiddruck proportional ist, oder wobei jedes der Vielzahl von Rückschlagventilen (302) mit einer anderen Öffnungsgröße ausgelegt ist und sich bei einem anderen Druckschwellenwert öffnet, so dass ein Fluidfluss durch das expandierbare Element (26) zu einem von der Pumpe aufgebrachten Fluiddruck proportional ist.

9. System (10) nach einem der vorhergehenden Ansprüche, wobei:
das Behandlungselement (23) ferner ein Abflusslumen zum Entfernen des Fluids aus dem expandierbaren Element (26) aufweist, und
eins von dem Zuflusslumen (27) und dem Abflusslumen, in Bezug auf das andere, näher an dem thermischen Element (30) positioniert ist.

10. System (10) nach einem der vorhergehenden Ansprüche, wobei das Rückschlagventil (302) dazu ausgelegt ist, sich bei einer Vielzahl von Öffnungsgrößen, die einer Vielzahl von Druckschwellenwerten entsprechen, zu öffnen, so dass ein Fluidfluss durch das expandierbare Element (26) zu einem von der Pumpe aufgebrachten Fluiddruck proportional ist.

11. System (10) nach einem der vorhergehenden Ansprüche, wobei:
das Behandlungselement (23) ferner ein Abflusslumen zum Entfernen des Fluids aus dem expandierbaren Element (26) aufweist, und
das Rückschlagventil (302) an einer Öffnung des Abflusslumens positioniert ist.

## Revendications

1. Système (10) d'ablation de tissu, le système (10) comprenant :
un dispositif de traitement comportant un élément de traitement (23), l'élément de traitement (23) comportant un élément expansible (26), un élément thermique (30), une lumière d'entrée (27) destinée à fournir un fluide à l'élément expansible (26), et un clapet antiretour (302) destiné à permettre, d'une manière pouvant être commandée, au fluide de s'écouler à partir de l'élément expansible (26) ; et
un ou plusieurs dispositifs de commande électroniques (33) couplés au dispositif de traitement et configurés pour :
commander une pompe pour fournir un volume du fluide à l'élément expansible (26) par l'intermédiaire de la lumière d'entrée (27) à une première pression ;
commander l'élément thermique (30) pour émettre de l'énergie afin d'effectuer une ablation sur un tissu cible pendant une période d'alimentation électrique ; et
pendant la période d'alimentation électrique, commander la pompe pour fournir le volume du fluide à l'élément expansible (26) par l'intermédiaire de la lumière d'entrée (27) à une seconde pression supérieure à la première pression, le clapet antiretour (302) étant configuré de telle sorte que la seconde pression provoque l'ouverture du clapet antiretour (302).

2. Système (10) selon la revendication 1, dans lequel le ou les dispositifs de commande électroniques (33) sont en outre configurés pour :
en réponse à l'expiration de la période d'alimentation électrique, commander l'élément thermique (30) pour arrêter l'émission d'énergie ; et
en réponse à l'expiration de la période d'alimentation électrique, commander la pompe pour fournir le volume de fluide à l'élément expansible (26) par l'intermédiaire de la lumière d'entrée (27) à la première pression.

3. Système (10) selon l'une quelconque des revendications précédentes, dans lequel le dispositif de traitement comporte en outre :
un capteur de température ; et
dans lequel le ou les dispositifs de commande électroniques (33) sont couplés au capteur de température et configurés en outre pour, pendant la période d'alimentation électrique :
recevoir un relevé de température en provenance du capteur de température ; et
commander la pompe en fonction du relevé de température.

4. Système (10) selon la revendication 3, dans lequel le ou les dispositifs de commande électroniques (33) sont en outre configurés pour :
comparer le relevé de température à une valeur seuil ; et
commander la pompe en fonction du relevé de température en commandant la pompe pour fournir le volume de fluide à l'élément expansible (26) par l'intermédiaire de la lumière d'entrée (27) à la seconde pression uniquement lorsque le relevé de température dépasse la valeur seuil ; et/ou dans lequel le ou les dispositifs de commande électroniques (33) sont en outre configurés pour :
en réponse au fait que le relevé de température dépasse une valeur seuil de température de fluide pendant la période d'alimentation électrique, commander l'élément thermique (30) pour arrêter l'émission d'énergie ; et/ou
dans lequel le ou les dispositifs de commande électroniques (33) sont en outre configurés pour :
recevoir un second relevé de température en provenance du capteur de température ;
déterminer une température prédite en fonction des premier et second relevés de température ;
comparer la température prédite à une seconde valeur seuil ; et
commander la pompe en fonction du relevé de température en commandant la pompe pour fournir le volume du fluide à l'élément expansible (26) par l'intermédiaire de la lumière d'entrée (27) à la seconde pression uniquement lorsque la température prévue dépasse la seconde valeur seuil.

5. Système (10) selon l'une quelconque des revendications 3 à 4, dans lequel le ou les dispositifs de commande électroniques (33) sont en outre configurés pour, pendant la période d'alimentation électrique :
recevoir un second relevé de température en provenance du capteur de température ;
déterminer un taux de variation en fonction des premier et second relevés de température ;
comparer le taux de variation à une seconde valeur seuil ; et
commander la pompe en fonction du relevé de température en commandant la pompe pour fournir le volume du fluide à l'élément expansible (26) par l'intermédiaire de la lumière d'entrée (27) à la seconde pression uniquement lorsque le taux de variation s'inscrit dans une plage prédéfinie de la seconde valeur seuil.

6. Système (10) selon l'une quelconque des revendications précédentes, dans lequel le clapet antiretour (302) est configuré pour, lorsqu'il est ouvert, augmenter un écoulement de fluide à travers l'élément expansible (26) tout en maintenant le volume du fluide à l'intérieur de l'élément expansible (26) ;
et/ou dans lequel le clapet antiretour (302) est positionné sur l'élément de traitement (23) de telle sorte que lorsque le clapet antiretour (302) est ouvert, une pression de fluide à l'intérieur de l'élément expansible (26) amène le fluide à s'écouler à travers le clapet antiretour (302) hors d'une extrémité distale de l'élément de traitement (23) ; et/ou dans lequel le clapet antiretour (302) est positionné sur une surface extérieure de l'élément expansible (26) de telle sorte que, lorsque le clapet antiretour (302) est ouvert, une pression de fluide à l'intérieur de l'élément expansible (26) provoque l'écoulement du fluide à travers le clapet antiretour (302) hors de l'élément de traitement (23).

7. Système (10) selon l'une quelconque des revendications précédentes, dans lequel l'élément de traitement (23) comporte une pluralité de clapets antiretour (302) positionnés sur l'élément de traitement (23) de telle sorte que lorsqu'un quelconque clapet de la pluralité de clapets antiretour (302) est ouvert, une pression de fluide à l'intérieur de l'élément expansible (26) amène le fluide à s'écouler hors d'une pointe distale de l'élément de traitement (23).

8. Système (10) selon la revendication 7, dans lequel chaque clapet de la pluralité de clapets antiretour (302) est configuré pour s'ouvrir à un seuil de pression différent, de telle sorte qu'un écoulement de fluide à travers l'élément expansible (26) soit proportionnel à une pression de fluide appliquée par la pompe, ou dans lequel chaque clapet de la pluralité de clapets antiretour (302) est configuré avec une taille d'ouverture différente et pour s'ouvrir à un seuil de pression différent, de telle sorte qu'un écoulement de fluide à travers l'élément expansible (26) soit proportionnel à une pression de fluide appliquée par la pompe.

9. Système (10) selon l'une quelconque des revendications précédentes, dans lequel :
l'élément de traitement (23) comporte en outre une lumière de sortie pour éliminer le fluide de l'élément expansible (26), et
la lumière d'entrée (27) ou la lumière de sortie est positionnée plus près, par rapport à l'autre, de l'élément thermique (30).

10. Système (10) selon l'une quelconque des revendications précédentes, dans lequel le clapet antiretour (302) est configuré pour s'ouvrir à une pluralité de tailles d'ouverture correspondant à une pluralité de seuils de pression, de telle sorte qu'un écoulement de fluide à travers l'élément expansible (26) soit proportionnel à une pression de fluide appliquée par la pompe.

11. Système (10) selon l'une quelconque des revendications précédentes, dans lequel :
l'élément de traitement (23) comporte en outre une lumière de sortie pour éliminer le fluide de l'élément expansible (26), et
le clapet antiretour (302) est positionné au niveau d'une ouverture de la lumière de sortie.
